# FASCICULE DE BREVET EUROPEEN

(11) **EP 1 785 154 B1**
(45) Date de publication et mention de la délivrance du brevet: **23.09.2009**
(21) Numéro de dépôt: 06291657.2
(22) Date de dépôt: 25.10.2006
(51) Int. Cl.: A61L 33/00

(54) **Matériau hémocompatible composite et son procédé d'obtention**
Hämokompatibles Kompositmaterial und sein Herstellungsprozess
Hemocompatible composite material and its process of manufacture

(30) Priorité: 10.11.2005 FR 0511430
(43) Date de publication de la demande: 16.05.2007
(73) Titulaire: CARMAT, 78140 Vélizy Villacoublay (FR)
(72) Inventeur: Capel, Antoine, 92140 Clamart (FR); Carpentier, Alain, 75116 Paris (FR); Melot, Marion, 75015 Paris (FR)
(74) Mandataire: Bonnetat, Christian

(56) Documents cités:
- EP-A2- 0 282 091
- EP-A2- 0 457 430
- DE-A1- 3 907 718
- US-A- 5 851 230

## Description

La présente invention concerne un matériau hémocompatible et son procédé d'obtention.

On sait que le sang est un tissu liquide vivant très sensible et qu'il est aisément altéré au contact de substances chimiques ou lors d'une exposition à des contraintes mécaniques, par exemple de cisaillement ; il coagule au contact de la plupart des matériaux inertes ou lors de stases. En réalité, il existe très peu de matériaux hémocompatibles et la plupart d'entre eux nécessitent la prise d'anticoagulants par un patient porteur d'un tel matériau hémocompatible.

On sait de plus (voir par exemple le document US-A-5 135 539) qu'il existe des prothèses cardiaques dans lesquelles les ventricules artificiels comportent des membranes souples, actionnées par des impulsions d'un fluide pour mettre le sang en mouvement. Dans ce cas, l'hémocompatibilité desdites membranes est particulièrement critique, du fait que les membranes sont mobiles et au contact d'un flux sanguin complexe et souvent turbulent.

Dans la technique, on connaît des matériaux sensiblement hémocompatibles qui sont soit synthétiques, soit d'origine biologique.

Les matériaux synthétiques sont généralement des élastomères de polyuréthane ou de silicone ; ils sont utilisés soit avec une surface lisse, afin de réduire les adhésions plaquettaires ou sanguines, soit avec une surface poreuse, afin de permettre l'adhésion d'une couche biologique apte à servir d'interface avec le sang. De tels matériaux synthétiques présentent de bonnes qualités de souplesse, d'imperméabilité et de déformabilité, mais nécessitent l'utilisation d'anticoagulants.

Les matériaux d'origine biologique sont des tissus d'animaux ou sont recomposés à partir de matière biologique, par exemple le collagène. Les tissus de nature animale doivent être fixés chimiquement (le plus souvent à l'aide de glutaraldéhyde) lorsqu'ils ont pour but d'être implantés dans le corps humain, afin d'éviter les réactions immunologiques. De tels matériaux biologiques ainsi traités ont généralement d'excellentes propriétés hémocompatibles et ne nécessitent d'ailleurs pas l'utilisation d'anticoagulants, mais ils ne sont absolument pas imperméables.

A contrario, les matériaux synthétiques, dits hémocompatibles et implantables, ont généralement des caractéristiques mécaniques et d'étanchéité intéressantes, mais ne sont tolérés dans le flux sanguin qu'au moyen d'une anticoagulation rigoureuse.

Pour pouvoir bénéficier des bonnes propriétés mécaniques et d'étanchéité des matériaux synthétiques et des bonnes caractéristiques d'hémocompatibilité des matériaux d'origine biologique, le document US-A-5 135 539 prévoit de superposer une membrane de matériau synthétique et une membrane d'origine biologique. Cependant, une telle disposition entraîne la formation d'une chambre intermédiaire entre lesdites membranes, qui peut être le siège d'infections ou de collections liquidiennes indésirables.

Le document WO 97/09006 décrit un matériau composite comportant un substrat synthétique fixé sur un tissu biologique assurant l'hémocompatibilité du produit.

L'objet de la présente invention est de remédier aux inconvénients précités et concerne un matériau hémocompatible souple, pouvant être déformé aisément et parfaitement imperméable.

A cette fin, selon l'invention, le matériau hémocompatible comportant un substrat synthétique, résistant et étanche, est remarquable en ce qu'il comporte un tissu biologique animal fixé chimiquement afin d'éviter des réactions immunologiques et rendu solidaire dudit substrat par une dispersion de la matière constitutive de ce dernier dans un solvant.

Ainsi, grâce à la présente invention, on obtient un matériau composite dont l'hémocompatibilité est assurée par le tissu biologique, alors que la résistance mécanique et l'étanchéité sont apportées par le substrat synthétique.

Bien que ledit substrat synthétique assure la résistance mécanique du matériau composite conforme à la présente invention, il est avantageux que le tissu biologique soit lui-même résistant. A cet effet, ce tissu biologique peut être constitué de péricarde animal, par exemple de péricarde bovin ou de tout autre tissu biologique.

Par ailleurs, il est avantageux que le substrat synthétique souple soit un élastomère. Cet élastomère peut être un élastomère de silicone ou un élastomère de polyuréthane. Cependant, pour des raisons d'étanchéité, il est préférable d'utiliser ce dernier élastomère pour réaliser ledit substrat synthétique souple.

La fabrication d'un matériau hémocompatible composite conforme à la présente invention comporte plusieurs étapes :
1. on commence par fixer chimiquement, de façon connue, le tissu biologique, de préférence constitué de péricarde animal, par tout produit approprié tel qu'un aldéhyde. Dans ce dernier cas, on utilise de préférence le glutaraldéhyde, par exemple à la concentration de 0,625 %. Une telle fixation chimique assure au tissu biologique, non antigénicité, stabilité chimique, biologique et physique, et notamment résistance aux variations de température et de contraintes mécaniques ;
2. ensuite, le tissu biologique est lyophilisé. Ce traitement a pour but d'une part de le déshydrater, ce qui est indispensable pour pouvoir le faire adhérer, mais aussi de conserver la structure tridimensionnelle dudit tissu biologique après déshydratation. En effet, lorsqu'un tissu biologique se déshydrate dans des conditions ordinaires, les fibres de collagène qui le constituent viennent au contact les unes des autres et il se crée des liaisons chimiques irréversibles rendant impossible la réhydratation ultérieure du tissu biologique. Pour éviter cet inconvénient, la lyophilisation permet d'immobiliser la structure du péricarde par congélation, puis de retirer l'eau à très basse pression par sublimation, donc sans permettre de mobilité et donc de réarrangement des fibres. Les deux paramètres, essentiels au contrôle de la lyophilisation, sont la cinétique et la déshydratation :
   ■ la cinétique doit être très lente -par exemple entre 2°C/heure et 8°C/heure- pour éviter tout risque de surfusion (fusion de l'eau entraînant une réhydratation locale) ;
   ■ le taux de déshydratation doit être au moins égal à 75%, de préférence de l'ordre de 78 à 80%. Un taux de déshydratation trop faible permettrait une certaine réhydratation des tissus et donc une certaine mobilité des fibres qui se traduirait par une perte de souplesse et une mauvaise réhydratation après une phase de stockage (même courte). Une déshydratation trop importante dénaturerait quant à elle le matériau biologique et entraînerait un rétreint important. Les matériaux biologiques sont en effet constitués d'eau liée (10% de matière sèche et 10% d'eau liée pour le péricarde) qui entre dans la constitution même du matériau et qu'il ne faut donc pas retirer.
   Pour améliorer encore la préservation de la structure tissulaire pendant la lyophilisation, le tissu biologique est d'abord traité pendant plusieurs jours par un glycol, avantageusement du polyéthylèneglycol, avant d'être lyophilisé. Le polyéthylèneglycol vient en effet créer des liaisons à basse énergie avec les différentes fibres de collagène et donc s'interposer entre ces fibres comme les barreaux d'une échelle. Pendant la lyophilisation, les différentes fibres ne peuvent donc pas venir interagir entre elles. Ces liaisons étant toutefois à basse énergie, le polyéthylèneglycol, bien que parfaitement biocompatible (pour certaines masses moléculaires, selon la pharmacopée européenne) est aisément rincé lors de la réhydratation ;
3. par ailleurs, sur ledit substrat synthétique souple, on dépose une couche d'une dispersion de la matière constitutive dudit substrat dans un solvant. Si, comme mentionné ci-dessus, ledit substrat est un élastomère de polyuréthane, ladite dispersion contient du polyuréthane biocompatible implantable dans un solvant dépendant du polyuréthane, qui peut être du diméthylacétamide. Cette dispersion, qui peut être déposée sur ledit substrat de toute manière connue (enduction, pulvérisation, etc ...) a pour objet de servir d'agent d'adhésion hémocompatible avec le tissu biologique. Puis, sur ladite couche d'agent d'adhésion hémocompatible, on applique ledit tissu biologique lyophilisé -qui s'imprègne de ladite dispersion- pour assurer l'adhésion mécanique de ladite membrane sur ledit substrat et obtenir ledit matériau composite ;
4. après quoi, on procède à l'élimination du solvant dudit agent d'adhésion hémocompatible, par exemple par séchage à chaud, séchage à chaud sous vide et/ou par extraction à chaud dans du sérum physiologique. De préférence, l'élimination du solvant est obtenue par une extraction lente à chaud (par exemple de l'ordre de 40°C), suivie d'une extraction sous vide et complétée par une extraction dans du sérum physiologique ;
5. enfin, si cela n'a pas été fait lors de la phase d'extraction du solvant, on réhydrate le matériau composite avec du sérum physiologique.

Dans le procédé conforme à l'invention, décrit ci-dessus, le traitement au polyéthylèneglycol et la lyophilisation permettent d'obtenir un tissu biologique sec, parfaitement réhydratable, sans altération dudit tissu et quasiment sans rétreint surfacique. Le collage par ancrage mécanique dudit tissu biologique au moyen de ladite dispersion, et non au moyen d'une colle (adhésion purement chimique), permet d'obtenir une très bonne adhésion mécanique du tissu sans dénaturation de ce dernier et sans ajout d'un produit supplémentaire (colle). La combinaison des différentes techniques d'extraction utilisées permet une extraction optimale des solvants, même lourds, sans dénaturer le tissu, soit par surchauffe, soit par prise d'humidité.

Le matériau composite ainsi obtenu allie les excellentes propriétés hémocompatibles du tissu biologique avec les propriétés mécanique et d'étanchéité du substrat synthétique. L'ensemble est de plus parfaitement souple et déformable.

## Revendications

1. Matériau hémocompatible, comportant un substrat synthétique, résistant et étanche, et un tissu biologique animal fixé chimiquement, afin d'éviter des réactions immunologiques,
**caractérisé en ce que** ledit tissu biologique animal est rendu solidaire dudit substrat par une dispersion de la matière constitutive de ce dernier dans un solvant, ladite matière constitutive imprégnant ledit tissu biologique animal.

2. Matériau hémocompatible selon la revendication 1,
**caractérisé en ce que** le tissu biologique est du péricarde animal.

3. Matériau hémocompatible selon l'une des revendications 1 ou 2,
**caractérisé en ce que** ledit substrat synthétique souple est un élastomère.

4. Matériau hémocompatible selon la revendication 3,
**caractérisé en ce que** ledit élastomère est un élastomère de polyuréthane ou de silicone.

5. Procédé pour la réalisation du matériau hémocompatible spécifié sous l'une quelconque des revendications 1 à 4,
**caractérisé en ce que,** après fixation chimique dudit tissu biologique animal :
- on traite pendant plusieurs jours ledit tissu biologique à l'aide d'un glycol, afin que ce dernier s'introduise entre les fibres dudit tissu biologique ;
- on lyophilise lentement ledit tissu biologique ainsi traité jusqu'à un taux de déshydratation au moins égal à 75% ;
- on colle ledit tissu biologique sur ledit substrat au moyen d'une dispersion de la matière constitutive dudit substrat dans un solvant ;
- on élimine ledit solvant ; et
- on procède à la réhydratation du matériau ainsi obtenu.

6. Procédé selon la revendication 5,
**caractérisé en ce que** le glycol utilisé est un polyéthylèneglycol.

7. Procédé selon l'une des revendications 5 ou 6,
**caractérisé en ce que** l'élimination dudit solvant est obtenue par une extraction lente à chaud et/ou une extraction sous vide et/ou une extraction dans du sérum physiologique.

8. Procédé selon l'une des revendications 5 à 7,
**caractérisé en ce que** la vitesse de lyophilisation est comprise entre 2°C/heure et 8°C/heure.

## Claims

1. A hemocompatible material comprising a strong and leaktight synthetic substrate and an animal biological tissue chemically fixed in order to prevent immunological reactions, **characterized in that** said animal biological tissue is tightly interlinked with said substrate by virtue of a dispersion of the constituent substance of said substrate in a solvent, said constituent substance impregnating said animal biological tissue.

2. The hemocompatible material as claimed in claim 1,
**characterized in that** the biological tissue is animal pericardium.

3. The hemocompatible material as claimed in either of claims 1 or 2, **characterized in that** said flexible synthetic substrate is an elastomer.

4. The hemocompatible material as claimed in claim 3,
**characterized in that** said elastomer is a polyurethane elastomer or a silicone elastomer.

5. A method for producing the hemocompatible material specified under any one of claims 1 to 4,
**characterized in that,** after chemical fixing of said animal biological tissue:
- said biological tissue is treated for several days with a glycol, in order for the latter to be introduced between the fibers of said biological tissue;
- said biological tissue thus treated is slowly freeze-dried until a degree of dehydration at least equal to 75% is reached;
- said biological tissue is bonded to said substrate by means of a dispersion of the constituent substance of said substrate in a solvent;
- said solvent is eliminated; and
- the material thus obtained is rehydrated.

6. The method as claimed in claim 5,
**characterized in that** the glycol used is a polyethylene glycol.

7. The method as claimed in either of claims 5 or 6,
**characterized in that** the removal of said solvent is obtained by means of slow hot extraction and/or extraction under vacuum and/or extraction in physiological saline.

8. The method as claimed in one of claims 5 to 7,
**characterized in that** the freeze-drying rate is between 2°C/hour and 8°C/hour.

## Patentansprüche

1. Hämokompatibles Material, umfassend ein widerstandsfähiges und dichtes synthetisches Substrat und ein tierisches biologisches Gewebe, das chemisch fixiert ist, um immunologische Reaktionen zu vermeiden,
**dadurch gekennzeichnet, dass** das tierische biologische Gewebe mit dem Substrat fest verbunden ist, und zwar durch eine Dispersion des Materials, das dieses Substrat bildet, in einem Lösemittel, wobei das bildende Material das tierische biologische Gewebe imprägniert.

2. Hämokompatibles Material nach Anspruch 1,
**dadurch gekennzeichnet, dass** das das biologische Gewebe ein tierischer Herzbeutel ist.

3. Hämokompatibles Material nach einem der Ansprüche 1 oder 2,
**dadurch gekennzeichnet, dass** das flexible synthetische Substrat ein Elastomer ist.

4. Hämokompatibles Material nach Anspruch 3,
**dadurch gekennzeichnet, dass** das Elastomer ein Polyurethan- oder Silikon-Elastomer ist.

5. Verfahren zur Herstellung des hämokompatiblen Materials, das in einem der Ansprüche 1 bis 4 spezifiziert wird,
**dadurch gekennzeichnet, dass** nach der chemischen Fixierung des tierischen biologischen Gewebes
- das biologische Gewebe während mehrerer Tage mit Hilfe eines Glykols behandelt wird, damit dieses zwischen die Fasern des biologischen Gewebes eindringt;
- das auf diese Weise behandelte biologische Gewebe langsam bis zu einem Dehydrierungsgrad von mindestens gleich 75 % gefriergetrocknet wird;
- das biologische Gewebe mittels einer Dispersion des Materials, welches das Substrat bildet, in einem Lösemittel an das Substrat geklebt wird;
- das Lösemittel entfernt wird; und
- eine Rehydrierung des auf diese Weise hergestellten Materials vorgenommen wird.

6. Verfahren nach Anspruch 5,
**dadurch gekennzeichnet, dass** das verwendete Glykol ein Polyethylenglykol ist.

7. Verfahren nach einem der Ansprüche 5 oder 6,
**dadurch gekennzeichnet, dass** die Entfernung des Lösemittels durch eine langsame Warmextraktion und/oder eine Extraktion im Vakuum und/oder eine Extraktion in physiologischer Salzlösung erfolgt.

8. Verfahren nach einem der Ansprüche 5 bis 7,
**dadurch gekennzeichnet, dass** die Geschwindigkeit der Gefriertrocknung zwischen 2°C/Stunde und 8°C/Stunde liegt.
